# EUROPEAN PATENT APPLICATION

(11) **EP 4 751 806 A1**
(43) Date of publication of application: **03.06.2026**
(21) Application number: 24216727.8
(22) Date of filing: 02.12.2024
(51) Int. Cl.: B01J 23/22, B01J 35/00, B01J 35/55, B01J 37/00, B01J 37/02, B01J 37/08, C07C 51/265, C07C 63/16, C07D 307/89

(54) **CATALYST AND PRE-CATALYST FOR PRODUCING PHTHALIC ANHYDRIDE**

(71) Applicant: BASF SE, 67056 Ludwigshafen am Rhein (DE)
(72) Inventor: WALSDORFF, Christian, 67056 Ludwigshafen am Rhein (DE); GAO, Wei, Shanghai, Shanghai 200137 (CN); RAUSCHKOLB, Michael, 67056 Ludwigshafen am Rhein (DE); GRAEF, Simon, 67056 Ludwigshafen am Rhein (DE); TRIMBORN, Florian, 67056 Ludwigshafen am Rhein (DE); CHU, Hiu Ping, Shanghai, Shanghai 200137 (CN); FENLON, Thomas, 67056 Ludwigshafen am Rhein (DE)
(74) Representative: Meissner Bolte Partnerschaft mbB

(57) **Abstract**

A pre-catalyst thermally convertible into a catalyst for producing phthalic anhydride by gas-phase catalytic oxidation of ortho-xylene and/or naphthalene with a molecular oxygen-containing gas comprises a support body, and coated on the support body, a coating composition. The coating composition comprises titanium dioxide; vanadium pentoxide or a vanadium compound thermally convertible to vanadium pentoxide; a binder polymer selected from polyvinyl esters, polyvinyl ester alkylene copolymers and polymers and copolymers of ethylenically unsaturated carboxylic acids such as acrylic acid, methacrylic acid and/or maleic acid, and salts and esters thereof; and a hydrophilic polymer selected from polyalkylene oxides, polyvinyl alcohol and starch. A catalyst obtained by thermal treatment of the pre-catalyst of the invention allows for a high yield and high selectivity in the production of phthalic anhydride.

## Description

The present invention relates to a catalyst for producing phthalic anhydride by gas-phase catalytic oxidation of ortho-xylene and/or naphthalene, a pre-catalyst therefor, and a process using the catalyst.

Phthalic anhydride is prepared industrially by catalytic gas-phase oxidation of o-xylene or naphthalene. Overviews of phthalic anhydride production are provided, e.g., by Dias et al. in "Synthesis of Phthalic Anhydride: Catalysts, Kinetics, and Reaction Modeling", Catalysis Reviews, 1997, 39:3, 169-207; Munck et al., "Oxidation of o-Xylene and Naphthalene to Phthalic Anhydride" in Industrial Arene Chemistry: Markets, Technologies, Sustainable Processes and Cases Studies of Aromatic Commodities, 2023, doi: 10.1002/9783527827992.ch39; and Richter et al. in "Oxidation of o-Xylene and Naphthalene to Phthalic Anhydride - Catalyst Development (Case Study)" in Industrial Arene Chemistry: Markets, Technologies, Sustainable Processes and Cases Studies of Aromatic Commodities, 2023, doi: 10.1002/9783527827992.ch40. Vanadium oxides on titania (anatase) as support material are used as heterogeneous catalysts. Promotors such as alkaline salts, antimony oxide or phosphoric acid are generally used as promotors. The catalytically active material is generally not used in pure form but as an active layer (coating) on preferably catalytically inert shaped materials such as steatite rings.

For process and economic reasons a nearly complete single-pass conversion of feed molecules is desirable. At the same time yield losses due to formation of intermediate and consecutive by-products should be limited. Main by-products are carbon oxides (CO and CO₂), which are formed by overoxidation, and in the case of naphthalene additionally as an unavoidable coupling product, and maleic anhydride formed by overoxidation. Intermediate products are often difficult to separate and may even in traces harm product quality such as colour. Main intermediate products are phthalide (from o-xylene) or naphthoquinone (from naphthalene).

The starting material is a mixture of a gas comprising molecular oxygen, for example air, and the o-xylene and/or naphthalene to be oxidized. The mixture is passed through a large number of parallel tubes located in a reactor (multi-tubular reactor, also known as a shell-and-tube reactor), with each of the tubes containing a bed of at least one catalyst. To control the temperature, the tubes are surrounded by a heat transfer medium, for example a salt melt (a.k.a. salt bath). Nevertheless, local temperature maxima (hot spots) tend to form in the catalyst bed, in which the temperature is higher than in the vicinity thereof. Excessive hot spots may favor secondary reactions such as total combustion of the starting material. Temperature profiles along the reaction tubes may also have an impact on the formation of undesirable by-products which can be separated from the reaction product only with difficulty, if at all. Care should be taken to ensure a homogeneous catalyst filling with very similar pressure drop and catalytic activity along each of the parallel reactor tubes.

The process is typically operated within the explosive regime and ignitions should be avoided. Therefore, hot-spot temperatures (maximum temperature achieved in the reactor tubes) should be controlled below 450 to 460 °C. Higher temperatures increase the risk of ignition or thermal run-away reactions, may disrupt the production process, damage the catalyst (deactivation and reduced catalyst life-time) and tend to reduce phthalic anhydride selectivity. In order to control hot-spot temperatures and the temperature profile along the reactor tubes, structured catalyst beds are employed. The temperature profile in turn has a strong impact on performance parameters such as conversion, selectivity and product quality. With modern catalysts, often four or more catalyst layers are successively filled in the reactor tubes on top of each other with specific activity of the individual catalyst layers generally increasing from reactor inlet to outlet.

Activity of individual catalyst layers can for example be defined by adjusting the amount of active mass in a specific reactor volume (such as coating more active mass material on the catalytically inert support bodies) or by adjusting the composition of the active mass itself (such as using different titania qualities, different amounts of vanadium oxide or different or different amounts of promotors). A gradual shift of catalytic activity in the reactor tubes from reactor inlet towards reactor outlet (deactivation) is observed with catalyst life-time (depending on process conditions in the order of 1 to 4 years) and reflected in corresponding shifts of temperature profiles. At start-of-run, it is generally desirable to be able to conduct the reaction at a relatively low temperature of the heat transfer medium and with a maximum hot-spot temperature in a part of the catalyst bed close to the tube inlet to provide for a sufficiently long life-time of the catalyst bed in operation. However, lower heat transfer medium temperatures may increase the amount of residual intermediate compounds such as naphthoquinone (in the case of naphthalene feed) or phthalide (in the case of o-xylene feed).

The oxidation reaction is highly exothermic and intra-particle heat- or mass-transport limitations should be avoided. This is reflected in the fact that coated catalysts, i.e., catalyst having a thin layer of active mass coated on a catalytically inert support body, have been exclusively used for many decades rather than shaped bodies made entirely of active mass. With a thickness of the coated layer of active mass in the order of up to a few 100 micro-meters the pore volume is located predominantly close to the geometric surface of the catalyst, allowing reactants to enter the pores and phthalic anhydride to pass out of the pores more quickly and avoiding long diffusion pathways within the catalyst body.

Catalysts for producing phthalic anhydride by gas-phase catalytic oxidation of ortho-xylene and/or naphthalene have been and still are the subject of extensive research. For example, catalysts with improved performance are described in Bin et al., "Performance of catalysts for preparation phthalic anhydride by technical naphthalene oxidation", Petrochemical Technology (2024), 53, 5, doi: 10.3969/j.issn.1000-8144.2024.05.003.

In order to facilitate the coating operation and provide a stable coating layer, an organic binder, such as a copolymer of vinyl acetate/vinyl laurate, vinyl acetate/ethylene or styrene/acrylate, may be incorporated into the aqueous suspension of the active components. The suspension is typically sprayed via nozzles onto the heated, fluidized support to provide a pre-catalyst. The binders serve primarily to reinforce the adhesion of the catalytically active composition on the support and to reduce attrition in the course of transport and charging of the catalyst. When the charged reactor is heated up and put into operation, the binders burn off within a short time in the molecular oxygen-containing gas atmosphere.

WO 98/37965 A1 describes a method for producing phthalic anhydride using a coated catalyst comprising titanium, vanadium and cesium. Optional auxiliary components used in the production of the coated catalyst include binders, pore formers and/or temporary activitiy dampening agents. In particular, the working examples describe spraying steatite rings with a suspension comprising, e.g., anatase, vanadyl oxalate, cesium sulfate, water and formamide. Formamide and its potential decomposition products are however detrimental to health and should be avoided in industrial processes.

WO 2023/16706 A1 describes the preparation of catalysts for the production of phthalic anhydride, wherein an organic binder such as Vinavil^{®} EVA 4612 is added to an aqueous slurry of milled metal oxides and metal precursors, and applying the slurry to an inert support.

CN 116060063 A descirbes a catalyst for preparing phthalic anhydride by oxidation of naphthalene, wherein metal compounds and precursors are provided in an aqueous slurry comprising formamide and a vinyl acetate / ethylene copolymer emulsion, and the slurry is applied to a carrier.

There is constant need for catalysts which enable an increase in productivity and economic viability. It is therefore an object of the present invention to provide an improved catalyst and pre-catalyst for producing phthalic anhydride by gas-phase catalytic oxidation of ortho-xylene and/or naphthalene.

The present invention provides a pre-catalyst thermally convertible into a catalyst for producing phthalic anhydride by gas-phase catalytic oxidation of ortho-xylene and/or naphthalene with a molecular oxygen-containing gas, the pre-catalyst comprising a support body, and coated on the support body, a coating composition comprising titanium dioxide; vanadium pentoxide or a vanadium compound thermally convertible to vanadium pentoxide; a binder polymer selected from polyvinyl esters, polyvinyl ester alkylene copolymers and polymers and copolymers of ethylenically unsaturated carboxylic acids such as acrylic acid, methacrylic acid and/or maleic acid, and salts and esters thereof; and a hydrophilic polymer selected from polyalkylene oxides, polyvinyl alcohol and starch.

The invention moreover provides a catalyst for producing phthalic anhydride by gas-phase catalytic oxidation of ortho-xylene and/or naphthalene with molecular oxygen, obtained by subjecting the pre-catalyst to thermal treatment.

It has been found that a catalyst obtained by thermal treatment of the pre-catalyst of the invention allows for a high yield and high selectivity in the production of phthalic anhydride.

Without wishing to be bound by theory, the inventors believe that the presence of the hydrophilic polymer in the coating composition allows for a fine distribution of the vanadium compound during the coating operation and/or retaining a fine distribution during the baking out of the binder during thermal treatment. Presumably, complexation of the transition metal species by the hydrophilic polymer occurs, which modifies the way in which deposition on the titania occurs. A fine distribution of the vanadium compound may improve catalyst performance. For example, the residual concentration of intermediates such as phthalides or naphthoquinone is reduced.

It is moreover believed that a catalyst obtained by thermal treatment of the pre-catalyst of the invention allows for a better control of temperature profiles in a multilayer catalyst bed and exhibits an improved life-time, in particular when it is used as catalyst layer adjacent to the inlet.

Moreover, the pre-catalysts of the invention show improved mechanical stability, in particular exhibiting improved adherence of the coating composition to the support body and reduced brittleness of the applied coating composition.

The coating composition is coated on the support body and is understood to form a coating layer on the support body. In the following, the coating composition and the coating layer are used synonymously where applicable.

The coating composition comprises a hydrophilic polymer selected from polyalkylene oxides, polyvinyl alcohol and starch, in particular polylalkylene oxides.

Suitable polyalkylene oxides include polyalkylene oxides obtained by polmerization of at least one alkylene oxide selected from the group of C₂- to C₁₀-alkylene oxides, preferably from the group of C₂- to C₅-alkylene oxides, such as ethylene oxide, propylene oxide, 1,2-butylene oxide, 2,3-butylene oxide, 1,2-pentene oxide or 2,3-pentene oxide. In one embodiment, the polyalkylene oxide is obtained by polmerization of at least one alkylene oxide selected from ethylene oxide, propylene oxide and 1 ,2-butylene oxide, in particular from ethylene oxide and propylene oxide. In a preferred embodiment, the polyalkylene oxide is polyethylene glycol.

The polyethylene glycol, depending on molar mass distribution may be liquid, semi-liquid or solid am ambient conditions.The polyethylene gylcol preferably has a molar mass distribution such that Mₙ (number average molecular weight) is at least 100 g/mol, more preferably at least 1,000 g/mol, most preferably at least 4,000 g/mol. The number average molecular weight Mₙ may be determined by various methods and is typically specified and reported for polyethylene glycol product grades by the vendor. In particular, the number-average molecular weight Mₙ may be determined by size exclusion chromatography (SEC) as follows: against polyethylene oxide standard 22000, in 100 mmol/L of sodium nitrate with 10% acetonitrile, at 40 °C/, flow rate 1.0 mL/min and triple detection (low-angle light scattering detector, refractive index detector and viscometry detector from Malvern-Viscotex) on an Ultrahydrogel 1000, 500, 250 column set from Waters Corp. USA with an injection volume of 100 µL.

Suitably, polyinvyl alcohol comprised in the coating composition has a number average molecular weight Mₙ of at least 100 g/mol, more preferably at least 500 g/mol, most preferably at least 1,000 g/mol.

Suitably, starch comprised in the coating composition has a number average molecular weight Mₙ of at least 500 g/mol, more preferably at least 1,000 g/mol, most preferably at least 5,000 g/mol.

In one embodiment, the coating composition comprises the hydrophilic polymer in an amount of 1 to 30 wt.-%, preferably 2 to 20 wt.-%, more preferably 5 to 15 wt.-%, relative to the total weight of the coating composition, as calculated after loss on ignition at 450 °C.

The coating composition moreover comprises a binder polymer selected from polyvinyl esters, polyvinyl ester alkylene copolymers, and polymers and copolymers of ethylenically unsaturated carboxylic acids such as acrylic acid, methacrylic acid and/or maleic acid, and salts and esters thereof, in particular polyvinyl esters and polyvinyl ester alkylene copolymers, most preferably polyvinyl ester alkylene copolymers.

Suitable polyvinyl esters include polyvinyl acetate, polyvinyl propionate, copolymers of vinyl acetate and vinyl laurate, and copolymers of vinyl acetate and vinyl maleate, in particular polyvinyl acetate or copolymers of vinyl acetate and vinyl laureate.

Suitable polyvinyl ester alkylene copolymers include copolymers of vinyl acetate and ethylene.

Polymers and copolymers of ethylenically unsaturated carboxylic acids such as acrylic acid, methacrylic acid and/or maleic acid, and salts and esters thereof are not particularly limited and may comprise essentially any such compounds known to be suitable as binders.

Preferably, binders are used in the form of an aqueous dispersion of the organic polymers or co-polymers. Such binder dispersions have a solids content (wt.-% by solids) of organic polymers or co-polymers of 5 to 95 wt.-%, preferably 20 to 80 wt.-% and more preferably 40 to 60 wt.-%.

In one embodiment, the coating composition comprises the binder polymer in an amount of 1 to 50 wt.-% by solids, preferably 5 to 40 wt.-% by solids, more preferably 10 to 30 wt.-% by solids, relative to the total weight of the coating composition, as calculated after loss on ignition at 450 °C.

In one embodiment, the coating composition comprises the hydrophilic polymer in an amount of 1 to 30 wt.-%, preferably 2 to 20 wt.-%, more preferably 5 to 15 wt.-%, and the binder polymer in an amount of 1 to 50 wt.-% by solids, preferably 5 to 40 wt.-% by solids, more preferably 10 to 30 wt.-% by solids, relative to the total weight of the coating composition, as calculated after loss on ignition at 450 °C.

The coating composition further comprises titanium dioxide and vanadium pentoxide and/or a vanadium compound thermally convertible to vanadium pentoxide. A vanadium compound thermally convertible to vanadium pentoxide includes any vanadium compound that is convertible to vanadium pentoxide by thermal treatment at a temperature of 350 to 450 °C, optionally under an oxidizing atmosphere. Suitable vanadium compounds include vanadium oxides and vanadyl carboxylates, in particular vanadyl oxalates. Examples of the vanadium compound include vanadyl compounds such as VOCI₃, VOSO₄, vanadyl oxalates and ammonium metavanadate. In a preferred embodiment, the vanadium compound is vanadyl oxalate. Vanadyl oxalates are preferably obtained by treating vanadium pentoxide (V₂O₅) with oxalic acid, more preferably in situ in the preparation of the coating composition.

In a preferred embodiment, the vanadium compound thermally convertible to vanadium pentoxide has a high aqueous solubility. For example, vanadyl oxalates typically exhibit high aqueous solubility.

In one embodiment, the coating composition comprises the vanadium pentoxide or vanadium compound thermally convertible to vanadium pentoxide in an amount of 1 to 15 wt.-%, preferably 2 to 10 wt.-%, more preferably 3 to 7 wt.-%, calculated as vanadium pentoxide, relative to the total weight of the coating composition, after loss on ignition at 450 °C. The coating composition, after loss on ignition at 450 °C, and thermal conversion of convertible vanadium compounds into vanadium pentoxide, is sometimes also referred to as "active mass" or "catalytically active material" in the catalyst.

In one embodiment, the coating composition comprises titanium dioxide in an amount of 82 to 99 wt.-%, preferably 85 to 97 wt.-%, more preferably 87 to 95 wt.-%, relative to the total weight of the coating composition, after loss on ignition at 450 °C.

Preferably, the titanium dioxide is in the anatase form. The titanium dioxide in the coating composition, i.e., prior to heat treatment of the pre-catalyst, preferably has an average BET surface area of 5 to 60 m²/g, more preferably 10 to 40 m²/g, most preferably 15 to 30 m²/g. The titanium dioxide used may consist of a single commercial titanium dioxide grade or a mixture of titanium dioxide grades. In the latter case, the value of the BET surface area may be calculated as the weighted mean of the contributions of the individual titanium dioxides. Alternatively, the BET surface area is determined directly from the mixture of titanium dioxides.

The coating composition may additionally comprise one or more further catalytically active compounds, also referred to as "dopants" or "promotors", selected from a cesium compound, a potassium compound, a phosphorous compound, a niobium compound, and an antimony compound.

Suitable cesium compounds include cesium oxides and cesium hydroxide, as well as cesium salts such as carboxylates, especially acetate, malonate or oxalate, carbonate, hydrogencarbonate, sulfate or nitrate.

Suitable potassium compounds include potassium oxide, potassium sulfate and potassium carbonate.

Suitable phosphorus compounds include phosphoric acid, phosphorous acid, hypophosphorous acid, ammonium phosphate or phosphoric esters, and in particular ammonium dihydrogenphosphate.

Suitable niobium compounds include niobium oxide.

Suitable antimony compounds include antimony oxides, especially antimony trioxide. In general, antimony trioxide with a mean particle size (maximum of the particle size distribution) of 0.1 to 10 µm is used. The source of the antimony oxide used is more preferably particulate antimony trioxide with a mean particle size of 0.5 to 5 µm, especially 1 to 4 µm.

In one embodiment, the coating composition comprises the further catalytically active compounds in an amount of 0.1 to 2.0 wt.-%, preferably 0.2 to 1.8 wt.-%, relative to the total weight of the coating composition, calculated after loss on ignition at 450 °C.

A multitude of other oxidic compounds may be present in small amounts in the catalytically active material, which, as promoters, influence the activity and selectivity of the catalyst, for example by lowering or increasing its activity. Examples of such promoters include alkali metal oxides besides those as mentioned above, such as lithium oxide and rubidium oxide, thallium(I) oxide, aluminum oxide, zirconium oxide, iron oxide, nickel oxide, cobalt oxide, manganese oxide, tin oxide, silver oxide, copper oxide, chromium oxide, molybdenum oxide, iridium oxide, tantalum oxide, arsenic oxide, cerium oxide, and tungsten oxide. They can be included in the coating composition as oxides or as compounds that are thermally convertible into the oxides.

In one embodiment, the pre-catalyst comprises the coating composition in an amount of 5 to 15 wt.-%, preferably 6 to 13 wt.-%, more preferably 7 to 11 wt.-%, relative to the total weight of the pre-catalyst composition, calculated after loss on ignition at 450 °C. The skilled person appreciates that this percentage corresponds to the percentage of the active mass in the catalyst.

The coating composition is coated on a support body. The support body is suitably catalytically inert. The support body is not particularly limited and includes essentially all support bodies found suitable in the prior art for coated catalysts for phthalic anhydride by gas-phase catalytic oxidation of ortho-xylene and/or naphthalene. The support body may comprise quartz (SiO₂), porcelain, magnesium oxide, tin dioxide, silicon carbide, rutile, alumina (Al₂O₃), aluminum silicate, cordierite, steatite (magnesium silicate), zirconium silicate, cerium silicate or mixtures of these support materials. The support is generally "non-porous". The term is to be understood in the sense of "non-porous apart from industrially ineffective amounts of pores", since it is technically unavoidable that a small number of pores be present in the support material which ideally should not comprise any pores. Advantageous support body materials are in particular steatite and silicon carbide, preferably steatite. In one embodiment, the support body comprises at least 80 wt.-% steatite, more preferably at least 90 wt.-% steatite or more than 99 wt.-% steatite. A certain degree of surface roughness may improve the adhesion (mechanical stability) of the coating layer.

The form of the support body is generally not critical for the inventive pre-catalysts and catalysts. For example, it is possible to use catalyst bodies in the form of spheres, rings, tablets, spirals, tubes, extrudates or spall. The dimensions of these catalyst supports correspond to those of catalyst supports typically used for preparing coated catalysts for the gas-phase partial oxidation of aromatic hydrocarbons.

The support material may be coated with the coating composition by any suitable state-of-the-art method. For example, a slurry of the coating composition can be sprayed onto the catalyst support in a heated coating drum. It is also possible to use fluidized bed coaters for the application of the coating composition to the support. A slurry of the coating composition is generally prepared by providing the components of the coating composition in the presence of a liquid. In one embodiment, the slurry of the coating composition is prepared in water. The slurry may also alternatively or additionally comprise an organic solvent, in particular an organic solvent selected from higher alcohols, polyhydric alcohols, e.g., ethylene glycol, 1,4-butanediol or glycerol, dimethylformamide, dimethylacetamide, dimethyl sulfoxide, N-methylpyrrolidone or cyclic ureas such as N,N'-dimethylethyleneurea or N, N'-dimethylpropyleneurea, or mixtures of these organic solvents with water. In one embodiment, the slurry of the coating composition is prepared in water. In another embodiment, the slurry of the coating composition does not comprise an organic solvent.

The layer thickness of the coating composition on the pre-catalyst is generally 0.02 to 0.2 mm, preferably 0.05 to 0.15 mm. Depending on the coating process and the shape and dimension of the support bodies, the layer thickness may vary around an average value along the surface of the support bodies.

The invention moreover relates to a catalyst for producing phthalic anhydride by gas-phase catalytic oxidation of ortho-xylene and/or naphthalene with molecular oxygen, obtained by subjecting the pre-catalyst as described above to thermal treatment. The thermal treatment is performed at a temperature in the range of 350 to 450 °C, preferably 370 to 410 °C, more preferably 380 to 400 °C.

As a result of thermal treatment of the precatalyst, the hydrophilic polymer and the binder polymer are generally at least partially baked out of the coating layers of the applied pre-catalyst bodies as a result of thermal decomposition and/or combustion. The thermal decomposition and/or combustion may commence already at temperatures below 400 °C, but is believed to be essentially complete at 450 °C. The thermal treatment is preferably conducted in situ within the gas-phase oxidation reactor, typically under a flow of a molecular oxygen-containing gas such as air, prior to feeding the o-xylene or naphthalene raw-material.

The catalysts of the invention are used for producing phthalic anhydride by gas-phase catalytic oxidation of ortho-xylene and/or naphthalene with a molecular oxygen-containing gas. For this purpose, the catalysts may be used alone or in combination with other catalysts of different activity, for example prior art catalysts based on vanadium oxide and anatase, in which case the different catalyst are generally arranged in separate sections (catalyst layers) along the reactor tubes of the multi-tube reactors.

In one aspect, the invention provides a multilayered catalyst bed for producing phthalic anhydride by gas-phase catalytic oxidation of ortho-xylene and/or naphthalene with molecular oxygen, comprising at least two successive layers of catalyst along the reactor tubes, each having an active composition comprising vanadium oxide and titanium dioxide, wherein at least one catalyst layer comprises a catalyst of the invention. In one embodiment, at least two catalyst layers differ in catalytic activity. The catalyst activity may be varied by adjusting the catalyst composition. For example, higher amounts of vanadium or lower amounts of cesium promoters allow for higher activity.

The invention moreover relates to a process for producing phthalic anhydride by gas-phase catalytic oxidation of ortho-xylene and/or naphthalene with molecular oxygen, comprising contacting ortho-xylene and/or naphthalene with molecular oxygen in the presence of a catalyst as described herein or in the presence of a multilayered catalyst bed as described herein.

In one embodiment, a reaction stream comprising ortho-xylene and/or naphthalene and molecular oxygen is passed through the catalyst layers of a multilayered catalyst bed as described herein, and wherein the catalyst activity is increased for at least one catalyst layer, relative to the previously passed catalyst layer. Preferably, the catalyst activity is increased for at least three successive catalyst layers, relative to the previously passed catalyst layer. In one embodiment, the catalyst activity is increased for each successive catalyst layer, relative to the previously passed catalyst layer.

To carry out the process of the invention, the catalysts or pre-catalysts may be charged into the reaction tubes of a tubular reactor which are heated up and thermostatted to the reaction temperature externally, for example by means of a salt melt. The initial heating up below the melting temperature of the salt melt is typically effected by using a pre-heated air flow. The reaction gas is passed through the catalyst bed prepared in this way at temperatures of from 200 to 470 °C and preferably from 300 to 450 °C and at an elevated pressure of generally from 0.1 to 2.5 bar, preferably from 0.3 to 1.5 bar. Typically, reactor tubes with an inner diameter of 20 to 30 mm and a length of 3 to 5 m are used, and 2 to 6, preferably 3 to 5 Nm³/h of the reaction gas are passed over each reactor tube. Nm³ refers to a normal cubic meter, being one cubic meter of gas at standard conditions of 0 °C and 1.01325 bar absolute.

The reaction gas supplied to the catalyst is generally obtained by mixing a gas which comprises molecular oxygen and, apart from oxygen, may also comprise suitable reaction moderators and/or diluents such as steam, carbon dioxide and/or nitrogen with the aromatic hydrocarbon to be oxidized, and the gas molecular oxygen-comprising gas may generally comprise from 1 to 100% by volume, preferably from 2 to 50% by volume and more preferably from 10 to 30% by volume of oxygen, from 0 to 30% by volume, preferably from 0 to 20% by volume of steam, and from 0 to 50% by volume, preferably from 0 to 1% by volume of carbon dioxide, remainder nitrogen. To obtain the reaction gas, the gas comprising molecular oxygen is generally supplied with from 30 to 150 g per m³ (STP), preferably with from 70 to 110 g per m³ (STP) of ortho-xylene and/or naphthalene. The molecular oxygen-comprising gas is particularly preferably air.

Generally, after the in-situ thermal treatment of the pre-catalyst in the rection tubes, during which the reaction tubes are maintained under a flow of air at a salt-bath temperature between 370 and 400 °C, metering of ortho-xylene and/or naphthalene to the reaction gas is commenced. Over the course of hours, days or more typically weeks, the salt-bath temperature is successively lowered and the feed of o-xylene and/or naphthalene to the oxygene containing gas is increased.

In another embodiment, the inventive pre-catalysts and catalysts can also be used in so-called postreactors as described for example in EP 2 027 102 B1. In such postreactors, which are generally operated at a temperature lower than the main reactor, the pre-catalyst or catalyst may comprise support bodies similar to the ones used in the main reactor or having a monolith ("honeycomb") shape, as described for example in EP 1 181 097 B1.

The invention is described in more detail by the accompanying drawings and the subsequent examples.

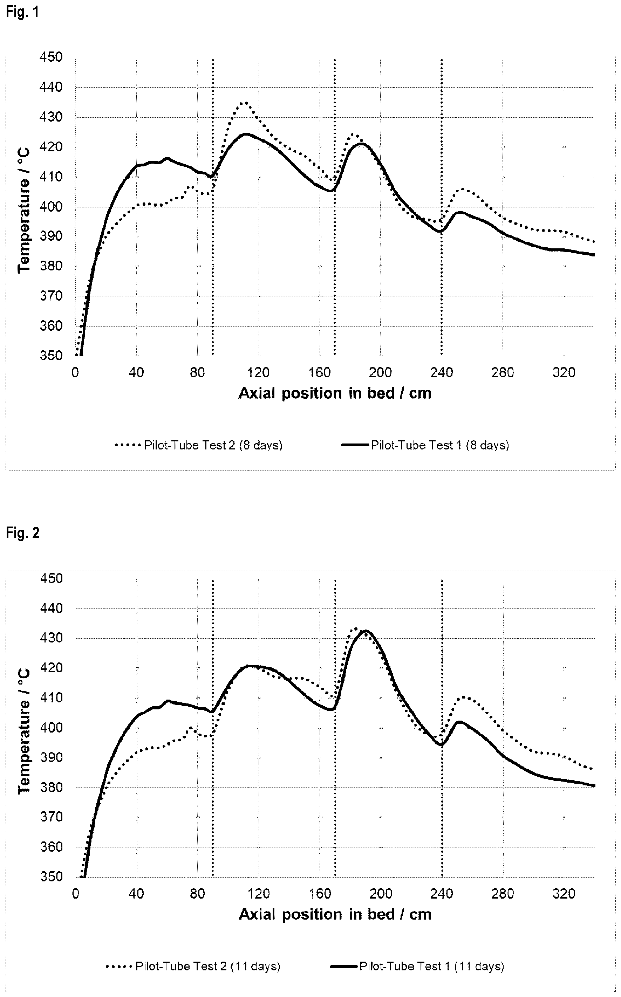

Fig. 1 shows the temperature profiles [°C] along the respective catalyst beds, as determined via multi-point thermo-element, relative to the axial position of the bed [cm], after 8 days of pilot-tube test 1 (solid graph line) and 8 days of pilot-tube test 2 (dotted graph line) as described below. The vertical dotted lines are indicative of the transition between the different types of catalysts (catalyst layers) within the respective catalyst bed.

Fig. 2 shows the temperature profiles [°C] along the respective catalyst beds, as determined via multi-point thermo-element, relative to the axial position of the bed [cm], after 11 days of pilot-tube test 1 (solid graph line) and 11 days of pilot-tube test 2 (dotted graph line) as described below. The vertical dotted lines are indicative of the transition between the different types of catalysts within the respective catalyst bed.

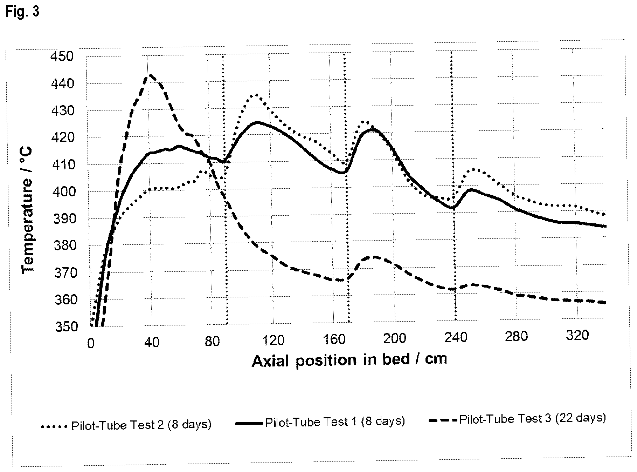

Fig. 3 shows the temperature profiles [°C] along the respective catalyst beds, as determined via multi-point thermo-element, relative to the axial position of the bed [cm], after 8 days of pilot-tube test 1 (solid graph line), 8 days of pilot-tube test 2 (dotted graph line) and 22 days of pilot-tube test 3 (dashed graph line) as described below, respectively. The vertical dotted lines are indicative of the transition between the different types of catalysts within the respective catalyst bed.

### Pre-Catalyst A

25.83 g of vanadium pentoxide were added to 700 g of stirred desalinated water. The suspension was heated up to about 45 °C and 58.6 g of oxalic acid were added. After the temperature had dropped by a few degrees, the suspension was heated to 60 °C and allowed to cool to room temperature.

615 g of desalinated water were added to the suspension. Under continued stirring, 0.73 g of ammonium dihydrogen phosphate, 7.18 g of cesium sulphate, 0.54 g of potassium sulfate, 574 g of titania (TiO₂ powder of anatase type with a BET surface area of about 20 m²/g) and 1.75 g of niobium oxide (Nb₂O₅) were added. 273 g of desalinated water were added, and the suspension was stirred at ambient temperature for 20 h.

To 870 g of the obtained suspension, 91.3 g of an aqueous dispersion based on a copolymer of vinyl acetate and ethylene (Vinnapas^{®} EZ 3010, 54 to 56 wt.-% by solids, Wacker) were added with stirring, followed by 24.2 g of polyethylene glycol (Pluriol^{®} E 8000, molecular weight Mₙ of 8,000 to 9,500 g/mol, BASF).

The obtained suspension was coated onto steatite rings (8 mm × 6 mm × 5 mm, outer diameter (OD) × inner diameter (ID) × height (HT)) in a coating apparatus as described in EP 1 670 575 B1 at an offgas temperature of about 90 °C to obtain pre-catalyst A with an active mass content of 9 wt.-% (weight of catalyst minus weight of steatite rings used, divided by weight of catalyst).

### Pre-Catalyst B (Reference)

Pre-catalyst B was prepared in the same manner as pre-catalyst A, except that no polyethylene glycol was added to the suspension.

### Pre-Catalyst C

Pre-catalyst C was prepared in the same manner as pre-catalyst A, except that no potassium sulfate was added to the suspension, and the amounts of vanadium pentoxide and oxalic acid were each increased by about 20%.

### Pre-Catalysts L1, L2 and L3 (Reference)

Pre-catalysts L1, L2 and L3 were prepared in the same manner as pre-catalyst A, except that no polyethylene glycol was added to the suspension. Moreover, the cesium content was reduced in the order of catalyst A > catalyst L1 > catalyst L2 > catalyst L3.

### Pre-Catalysts L1a, L2a and L3a (Reference)

Pre-catalysts L1a, L2a and L3a were prepared in the same manner as pre-catalysts L1, L2 and L3, respectively, except that neither potassium sulfate nor polyethylene glycol was added to the suspension.

### Pilot-Tube Test 1 - Pre-Catalyst A

A gas-phase catalytic oxidation of naphthalene for producing phthalic anhydride was carried out using a reactor tube (29 mm inner diameter) comprising a central thermowell along the reactor tube axis. The thermowell had an outer diameter of 10 mm and was fitted with a multi-point thermo-element continuously monitored by a Siemens process control system. For on-line gas chromatography (GC) analytics, small streams of reactor inlet and outlet flow were continuously and automatically taken and monitored.

The reactor tube was installed in a container of molten salt (salt bath). The salt bath temperature (SBT) was automatically controlled and continuously monitored.

The reactor tube was charged with a structured pre-catalyst bed as follows (in the direction from reactor inlet to reactor outlet):

| | |
|---|---|
| 15 cm | steatite slit rings 7 mm × 4 mm × 4 mm (OD × ID × HT) |
| 90 cm | Pre-Catalyst A |
| 80 cm | Pre-Catalyst L1 |
| 70 cm | Pre-Catalyst L2 |
| 100 cm | Pre-Catalyst L3 |

The reactor was closed, heated under airflow to a SBT of 390 °C, and maintained at this temperature for 12 h to thermally convert the pre-catalysts to catalysts and obtain a structured catalyst bed. Over the course of a few days, the airflow feed was loaded with increasing concentrations of naphthalene and the SBT lowered in small steps. Naphthalene of 99.2 wt.-% purity was used. The reactor inlet and outlet gas flow were continuously monitored by on-line GC.

### Pilot-Tube Test 2 - Pre-Catalyst B

The pilot-tube test was performed in the same manner as pilot-tube test 1, except that pre-catalyst B was used instead of pre-catalyst A.

### Pilot-Tube Test 3 - Pre-Catalyst C

The pilot-tube test was performed in the same manner as pilot-tube test 1, except that pre-catalyst C was used instead of pre-catalyst A, and pre-catalysts L1a, L2a and L3a were used instead of pre-catalysts L1, L2 and L3, respectively.

Data from the pilot-tube tests is shown in the table below.

| Pilot-Tube Test | 1 | | 2 | | 3 |
|---|---|---|---|---|---|
| Catalyst | A/L1/L2/L3 | | B/L1/L2/L3 | | C/L1a/L2a/L3a |
| Time on Stream [days] | 8 | 10 | 8 | 11 | 22 |
| Salt Bath Temperature [°C] | 378 | 374 | 378 | 374 | 350 |
| Airflow Feed Rate [Nm³/h] | 3.78 | 4.74 | 3.75 | 4.74 | 3.75 |
| Naphthalene Load [g/Nm³/h] | 80.0 | 80.5 | 81.0 | 81.0 | 80.2 |
| Naphthoquinone [wt.-%] * | 0.34 | 0.64 | 0.96 | 0.97 | 0.56 |
| Phthalic Anhydride Yield [wt.-%] ** | 101.54 | 103.61 | 99.64 | 99.78 | 103.34 |

| | | | | | |
|---|---|---|---|---|---|
| * in the phthalic anhydride raw-product ** the phthalic anhydride yield is reported in terms of weight percent, referring to the mass flow of phthalic anhydride formed per mass flow of naphthalene in the reactor feed. | | | | | |

It is evident that catalysts A and C allow for high yields of phthalic anhydride at high selectivity. In particular, it is evident that catalyst A allows for a higher yield of phthalic anhydride at higher selectivity than catalyst B. In direct comparison with catalyst B, inventive catalyst A shows a more desirable hot-spot profile, indicating a higher degree of catalyst activity in the first catalyst layer adjacent to the reactor inlet, as is evident from Fig. 1 and Fig. 2. Inventive catalyst C has been derived from inventive catalyst A to provide an embodiment with increased activity to provide a further improved temperature profile.

From Fig. 3, it is evident that catalyst C allows for a particularly favorable temperature profile with a hot-spot temperature in the first catalyst layer which is higher than in any of the subsequent catalyst layers, as well as allowing for a relatively low temperature of the salt bath while maintaining suitably low concentrations of naphthoquinone.

## Claims

1. A pre-catalyst thermally convertible into a catalyst for producing phthalic anhydride by gas-phase catalytic oxidation of ortho-xylene and/or naphthalene with a molecular oxygen-containing gas, the pre-catalyst comprising a support body, and coated on the support body, a coating composition comprising titanium dioxide; vanadium pentoxide or a vanadium compound thermally convertible to vanadium pentoxide; a binder polymer selected from polyvinyl esters, polyvinyl ester alkylene copolymers and polymers and copolymers of ethylenically unsaturated carboxylic acids such as acrylic acid, methacrylic acid and/or maleic acid, and salts and esters thereof; and a hydrophilic polymer selected from polyalkylene oxides, polyvinyl alcohol and starch.

2. The pre-catalyst according to claim 1, wherein the hydrophilic polymer is selected from polyalkylene oxides.

3. The pre-catalyst according to claim 2, wherein the hydrophilic polymer is polyethylene glycol, preferably polyethylene glycol with a number average molecular weight Mₙ of at least 500 g/mol.

4. The pre-catalyst according to any one of the preceding claims, wherein the binder polymer is selected from polyvinyl esters and polyvinyl ester alkylene copolymers, in particular polyvinyl ester alkylene copolymers.

5. The pre-catalyst according to claim 4, wherein the binder polymer is a copolymer of vinyl acetate and ethylene.

6. The pre-catalyst according to any one of the preceding claims, wherein the hydrophilic polymer is polyethylene glycol and the binder polymer is a copolymer of vinyl acetate and ethylene.

7. The pre-catalyst according to any one of the preceding claims, wherein the coating composition comprises 1 to 30 wt.-% of the hydrophilic polymer and 1 to 50 wt.-% of the binder polymer, relative to the total weight of the coating composition, as calculated after loss on ignition at 450 °C.

8. The pre-catalyst according to any one of the preceding claims, wherein the support body is an essentially non-porous support body.

9. The pre-catalyst according to any one of the preceding claims, wherein the titanium dioxide has an average BET surface area in the range of 5 to 60 m²/g, preferably 10 to 40 m²/g.

10. A catalyst for producing phthalic anhydride by gas-phase catalytic oxidation of ortho-xylene and/or naphthalene with molecular oxygen, obtained by subjecting the pre-catalyst according to any one of the preceding claims to thermal treatment.

11. The catalyst according to claim 10, wherein the thermal treatment is performed at a temperature in the range of 350 to 450 °C, preferably 370 to 410 °C, more preferably 380 to 400 °C, preferably under flow of a molecular oxygen-containing gas such as air.

12. A multilayered catalyst bed for producing phthalic anhydride by gas-phase catalytic oxidation of ortho-xylene and/or naphthalene with molecular oxygen, comprising at least two layers of catalyst, each having an active composition comprising vanadium oxide and titanium dioxide, wherein at least one catalyst layer comprises a catalyst in accordance with claim 10 or 11.

13. The multilayered catalyst bed according to claim 12, wherein at least two catalyst layers differ in catalytic activity.

14. A process for producing phthalic anhydride by gas-phase catalytic oxidation of ortho-xylene and/or naphthalene with molecular oxygen, comprising contacting ortho-xylene and/or naphthalene with molecular oxygen in the presence of a catalyst according to claim 10 or 11 or in the presence of a multilayered catalyst bed according to claim 12 or 13.

15. The process according to claim 14, wherein a reaction stream comprising ortho-xylene and/or naphthalene and molecular oxygen is passed through the catalyst layers of a multilayered catalyst bed according to claim 13, and wherein the catalyst activity is increased for at least one catalyst layer, relative to the previously passed catalyst layer.
